# EUROPEAN PATENT SPECIFICATION

(11) **EP 4 415 620 B1**
(45) Date of publication and mention of the grant of the patent: **04.11.2026**
(21) Application number: 22805789.9
(22) Date of filing: 13.10.2022
(51) Int. Cl.: A61B 5/22, A61B 5/00

(54) **ASSESSMENT OF HAMSTRING INJURY**
BEURTEILUNG VON HAMSTRING-VERLETZUNGEN
ÉVALUATION D'UNE LÉSION DES MUSCLES ISCHIO-JAMBIERS

(30) Priority: 13.10.2021 GB 202114621
(43) Date of publication of application: 21.08.2024
(73) Proprietor: Mcintyre, Martin, Castlebar Co. Mayo, F23YK37 (IE)
(72) Inventor: Mcintyre, Martin, Castlebar Co. Mayo, F23YK37 (IE)
(74) Representative: Tomkins & Co
(86) International application number: PCT/EP2022/078523
(87) International publication number: WO 2023/062133

(56) References cited:
- EP-B1- 3 124 085
- WO-A1-2014/032072
- WO-A1-2022/003207
- AU-A1- 2012 388 708
- AU-A1- 2017 204 276
- AU-A1- 2020 308 341
- CN-A- 115 253 189
- CN-A- 115 253 190
- KR-A- 20120 041 896
- US-A- 5 085 225
- US-A1- 20240 016 413
- US-A1- 20240 374 219
- US-B1- 6 468 188
- PATRÍCIA DIAS PANTOJA ET AL: "Effect of weighted sled towing on sprinting effectiveness, power and force-velocity relationship", PLOS ONE, 1 January 2018 (2018-01-01), United States, pages e0204473, XP055551916, Retrieved from the Internet <URL:https://www.researchgate.net/profile/Jean-Benoit_Morin/publication/329529013_A_new_testing_and_training_device_for_hamstring_muscle_function/links/5c0e44b8299bf139c74dd579/A-new-testing-and-training-device-for-hamstring-muscle-function.pdf> DOI: 10.1371/journal.pone.0204473
- TROY BLACKBURN J ET AL: "Influences of hamstring stiffness and strength on anterior knee joint stability", CLINICAL BIOMECHANICS, ELSEVIER, AMSTERDAM, NL, vol. 26, no. 3, 14 October 2010 (2010-10-14), pages 278 - 283, XP028164079, ISSN: 0268-0033, [retrieved on 20101020], DOI: 10.1016/J.CLINBIOMECH.2010.10.002
- GIACOMO JEAN-PATRICK ET AL: "A new testing and training device for hamstring muscle function A new testing and training device for hamstring muscle function", 1 December 2018 (2018-12-01), pages 1 - 4, XP093018733, Retrieved from the Internet <URL:https://sportperfsci.com/wp-content/uploads/2018/12/SPSR47_Morin_181203_final.pdf> [retrieved on 20230130]

## Description

### Field of the Invention

The present invention relates to a device and associated method for measuring hamstring function.

### Background to the Invention

The hamstrings are composed of the three posterior muscles of the thigh and include the semi membranous (SM), semi tendinous (ST), Biceps Femoris long and short heads (BFlh and BFsh). They are biarticular in nature in which they extend the hip and flex the knee.

The BF (239+/-49cm³) has usually the largest muscle volume followed by the SM (184+/-48cm³) and ST (163+/-65cm³). They can also be measured via (1) anatomical cross sectional area (ACSA), area of tissue perpendicular to its longitudinal axis or (2) physiological cross sectional area (PCSA) area of tissue perpendicular to its longitudinal axis of the fascicles and (3) muscle volume product of total muscle ACSA. Anatomical cross sectional area has been determined from MRI with SM (15.33+/-1.85) having the largest, followed by the BFlh (14.54+/-2.32), ST (13.06+/- 2.37), BFsh (8.38+/-1.11) at 77%, 58%, 47% and 77% of total muscle length respectively. The estimated average total muscle physiological cross sectional area from cadaver studies is greatest in the BFlh (12.68+/-2.58cm), SM (18.21+/-4.21), ST (5.39+/-1.59cm) and BFsh (5.03+/- 0.69cm).

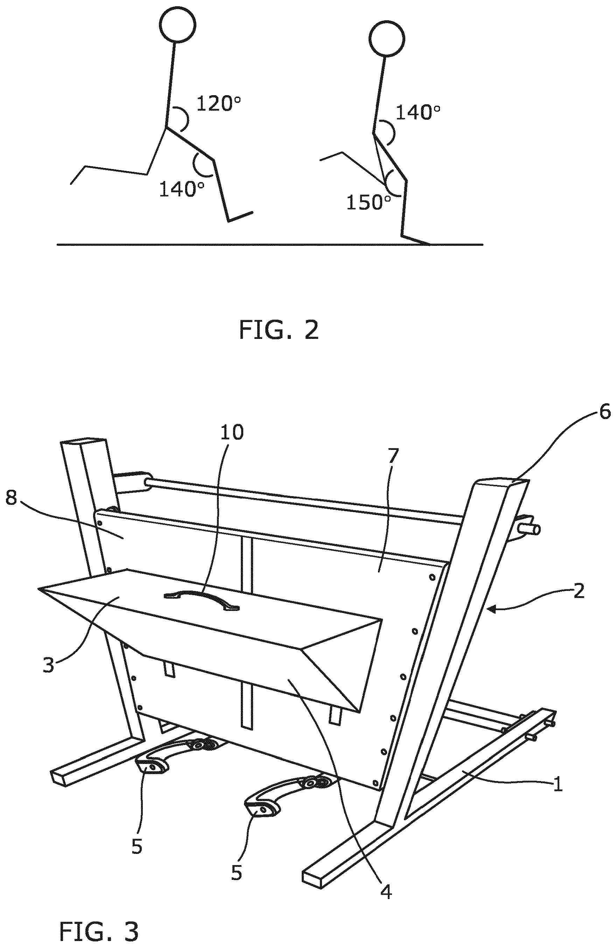

They have all unique tendon morphology with the SM the most robust tendon with the greatest cross sectional area (0.86cm²), largest proximal tendon (31.9cm), free tendon (11.1cm) and also has the largest muscle tendon junction (20.8cm). The BFlh and ST separate 9-10cm distal to their origins and are not as robust with a cross sectional area of 0.47cm², proximal tendons of 27.1 and 12.9cm, proximal free tendons of 6.3 and 11.2cm and finally muscle tendon junction lengths of 20.6 and 11.7cm, respectively (Figure 2.4). These unique characteristics predispose to hamstring strain injury (HSI) evident within all sports but particularly so within sports involving running and particularly those at high speed.

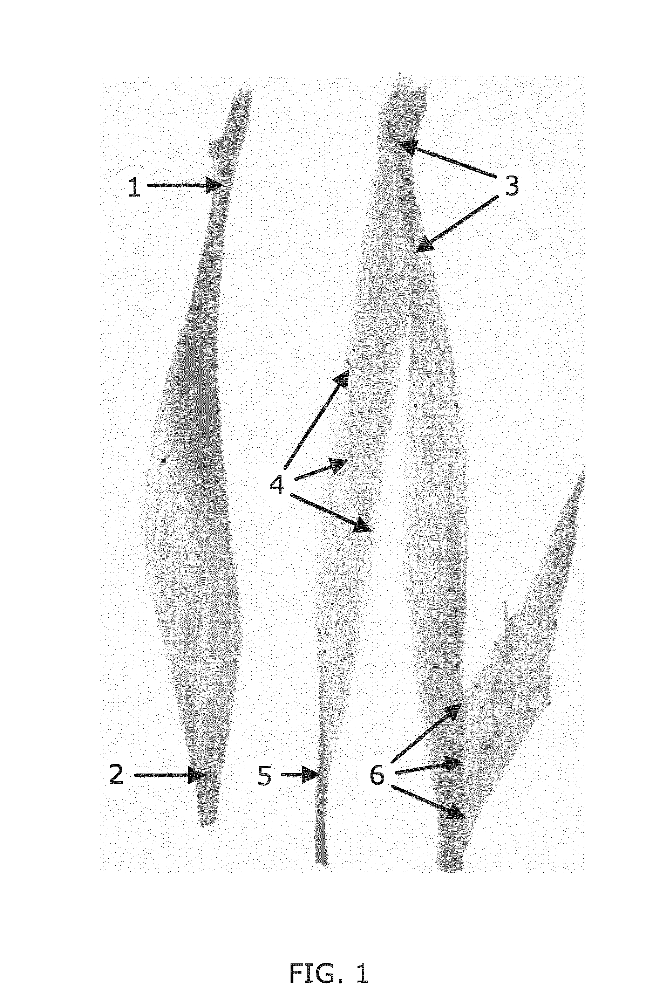

Hamstring anatomy is shown in Figure 1 in which (1) is the Proximal tendon of the SM muscle, (2) is the distal tendon of the SM muscle, (3) is the conjoined tendon of the ST and the BFlh, (4) is the tendinous inscription (raphe) of the ST muscle, (5) is the distal tendon of the ST muscle, (6) is the common distal tendon of the long and short head of the BF muscle.

HSI is a common injury within many sports consisting of 12% of all injuries in elite soccer, 15% in rugby union, 16%-17% in Australian Football, 12-29% in Gaelic football and 17% in Hurling. The situation within field sports is not improving with a twofold increase in HSI from 2008/2011 to 2012/2015. While in soccer a time trend analysis has presented with an annual average 2.3% increase in HSI over a 13 year period. There is a high recurrence rate (36-47 %) in Gaelic football with 44% of injuries re-occurring with the first 12 months of sport with over 20% of all injuries taking more than 28 days to recover from and it is also debated whether injury interventions are having any effect on injury rates.

It is important to consider the mechanism of injury and also acknowledge the physiological strain upon players, given the physical nature of the sport with 73% of all hamstring injuries occurring whilst running, sprinting (14%-26.8%), turning (12%), landing (7.1%) and kicking (4.5%). The higher incidence of injury in running can be linked to high speed in field sports of up to 39km/hr and even faster in athletics of 44.72km/hr by top sprinters, while the forces exhibited in which average peak hamstring forces can range between 2,880N-4,160N and average peak negative work 112-208J for a 80kg athlete when running at 80-100% of maximal speed.

There is a wide consensus that HSI is likely to occur in either late swing phase or early stance or possibly even in the transition from late swing to early stance as illustrated in Figure 2.

Hamstrings injuries are currently assessed via handheld dynamometers, isokinetic dynamometers, the Nordbord Hamstring Testing System (available from Vald Performance, Australia) and the Hamsting Solo device (available from HD Sports Performance, Ireland). These devices measure the concentric and eccentric strength of the hamstrings in various positions and have various procedures and protocols in place to do so but are device dependant. There is therefore a need to measure isometric strength and to detect deficits within the muscle.

Troy Blackburn J et al: "Influences of hamstring stiffness and strength on anterior knee joint stability" discloses a study on whether factors which limits excessive anterior tibial translation may reduce anterior cruciate ligament injury risk.

EP3124085B1 discloses a hamstring exercise machine for a user, comprising a frame, a knee support, a descent assistance system, and ankle restraints.

Giacomo Jean-Patrick et al: "A new testing and training device for hamstring muscle function" discloses a novel field device designed to improve hamstring muscle function via movement patterns closer to those observed when hamstring injuries typically occur. AU2012388708A1 discloses an apparatus for use in assessing strength of at least one knee flexor muscle of a subject, the apparatus including a support, two securing members, each securing member securing a respective lower leg of the subject in a position that in use is substantially fixed relative to the support and at least one sensor, which in use senses a force indicative of the strength of the at least one knee flexor muscle in at least one leg of the subject while the subject performs an eccentric contraction of the at least one knee flexor muscle.

### Object of the Invention

The object of the invention is to provide a device and a method that measures the isometric strength of the hamstring in a more novel and unique joint position in order to measure isometric strength and to detect deficits within the muscle.

With the late swing phase and early stance phase in mind, another object of the invention is to provide two tests 1) a Bilateral test and 2) a Unilateral test for hamstring function. These tests are unique due to the testing position of 30 degrees of knee flexion (which is equivalent to 150 degrees of knee extension) for both tests with also the introduction of 20 degrees of hip extension in the non-tested (contralateral) limb in the unilateral test. Another object is to provide teste which can be used to:-
1) Predict previous injury
2) Risk for future injury
3) Classify muscle grading following injury
4) Control return to play following injury.

### Summary of the Invention

According to the present invention there is provided a device according to claim 1 for the measurement of hamstring function comprising a base adapted to sit on the ground, a frame extending vertically from the base having two slots, a thigh support element having an inclined surface inclined at 27 to 33° to the horizontal adjustably mounted on the frame such that the element can slide up and down on the frame within the two slots and be locked in position via two lock wheels and at least one hook shaped element which can be fastened around the ankle of a user, the hook shaped element being mounted adjacent the base of the device, each of the hook shaped elements comprising at least one force sensor which can sense the force applied about the knee through the ankle by one of the hamstrings of a user pushing their thighs against the a thigh support element, and which can transmit the force which is sensed to a device which can record and measure the force. The force about the knee is measured through the ankle by the sensor.

The device which can record and measure the force may be a separate device.

Suitably when the user applies the force of the hamstrings with the thighs, the knees are in 150 ° extension (also expressed as 30 degrees knee flexion). Alternatively one limb can push and the non-tested limb can be in about 20 ° hip extension.

Preferably the frame comprises a planar element which is mounted substantially vertically on the frame. The planar element may have a front face and a rear face, the front face being the face which faces the user when the device is in use. The thigh support element may be mounted on the front face of the planar element. Suitably the inclined surface of the thigh support element is inclined at a 27 to 33°, preferably at a 29 to 31°, more preferably at a 30° angle to the horizontal such that its slopes away from the user towards the base of the device. This angle places the hamstrings in a long lever position for isometric testing in the bilateral protocol and the contralateral hip is placed in extension at a 10-30° angle for the unilateral protocol. The inclined surface may be provided with padded material.

Preferably the force sensor is a loadcell which is a transducer which converts force into a measurable electrical output. The electrical output may be recorded and analysed by a software platform. The electrical output may be transmitted to the software platform by Wi-Fi.

Suitably the device is provided with two hook shaped elements, one for each ankle of the user. Preferably the hook shaped element can be fastened around the ankle of the user. This may be achieved by means of a strap. The hook shaped element may be made of a plastics material. Suitable hook shaped elements are commercially available from VALD Performance, Australia.

Preferably the devices providing with a handle that the user can grasp during use of the device. The handle may take the form of a bar along the upper, in use position, of the device or it may take the form of two handgrips, one positioned on either side of the device, towards the upper in use position.

The device may be provided with a seat or bench on which the subject being tested may sit. Preferably the height of the seat or bench is adjustable, to accommodate different heights of test subjects.

The prior art device available from Vald Performance measures eccentric strength while performing an exercise called a Noric fallout. The device of the present invention measures isometric strength in which there is no movement at the unique joint positions previously discussed. It has been determined that the device and method of the invention is a more sensitive method of detecting injury to the hamstrings. The main advantages of these isometric tests is 1) the Iso_{BI} assesses muscle function in a long lever 150° position, whereas the Nordic fallout breakpoint occurs at 103⁰-126⁰ and as a result does not assess muscle function in long lever positions. The Iso_{UNI} may isolate the affected muscle group and preclude any crossover between limbs which occurs in bilateral testing. In addition, the placement of the contralateral hip into extension may simulate the mechanism of injury in which the contralateral hip is in extension during late swing phase/early stance.

Also disclosed herein is a method of measuring bilateral hamstring function according to claim 11 comprising sensing and measuring the force applied by a user pushing their thigh against a thigh support element having an inclined surface inclined at 27 to 33° to the horizontal such that its slopes away from the user in a downward direction.

In a still further aspect the invention provides a method of measuring unilateral hamstring function according to claim 12 with the tested leg comprising sensing and measuring the force applied by a user pushing their thighs against a thigh support element having an inclined surface inclined at 27 to 33° to the horizontal with the non-tested leg in 10-30° of hip extension. The inclined surface slopes away from the user in a downward direction.

In both methods the inclined surface is inclined at a 27 to 33°, preferably at a 29 to 31° angle, more preferably at a 30° angle to the horizontal such that its slopes away from the user in a downward direction.

Also in both methods the force about the knee is measured through the ankle by a sensor so that the force applied through the knee can be measured. No method encompassing determining hamstring injury or the risk of injury is forming part of the claims.

### Brief Description of the Drawings

Figure 1 shows the hamstring anatomy,
Figure 2 shows Late Swing and Early Stance phases of human movement,
Figure 3 is a perspective view of the device of the invention from the front,
Figure 4 is an end view from above of the device of the invention,

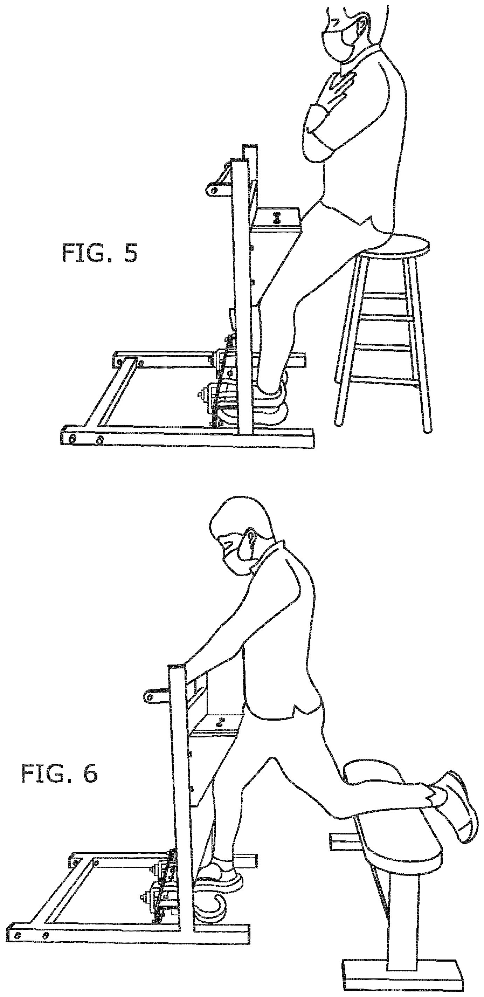

Figure 5, shows the device of the invention set up in the bilateral test position,
Figure 6, shows the device of the invention set up in the unilateral test position,
Figure 7 is an end view of the device of the invention from one side,
Figure 8 is a perspective view from the rear of the device of the invention, and
Figure 9 is a perspective view of a second embodiment of the device of the invention..

### Detailed Description of the Drawings

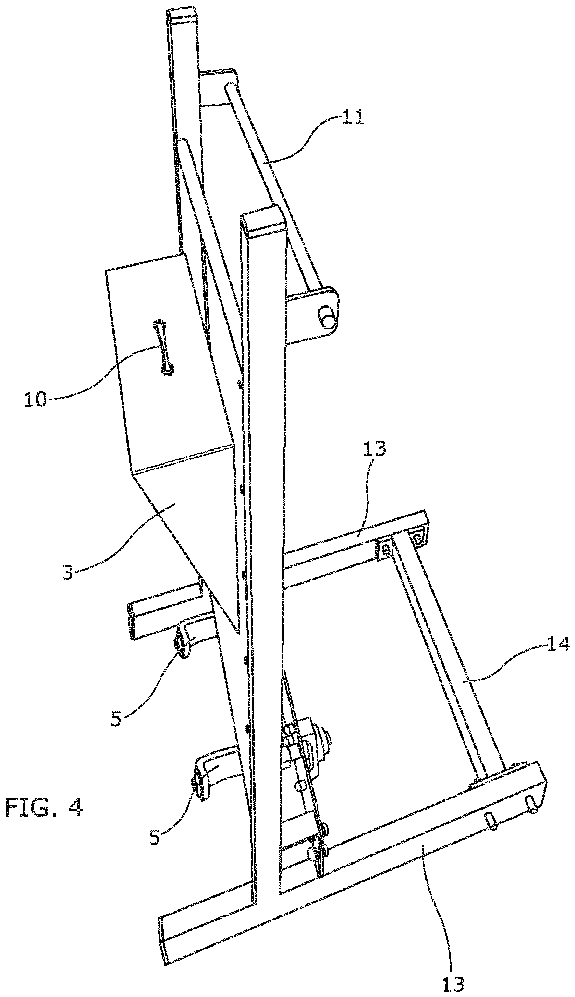

As shown in Figures 3 and 4 the device for the measurement of hamstring function comprises a base (1) adapted to sit on the ground, a frame (2) which extends vertically from the base, a thigh support element (3) having an inclined surface (4) which is adjustably mounted on the frame (2) and two hook shaped elements (5) mounted adjacent the base (1) of the device. The hook shaped elements (5) which wrap around the ankles of the user when the device is being used. The hook shaped elements (5) comprise at least one force sensor which is not visible in the figure. The device is connectable with a means of recording and analysing the force which is sensed.

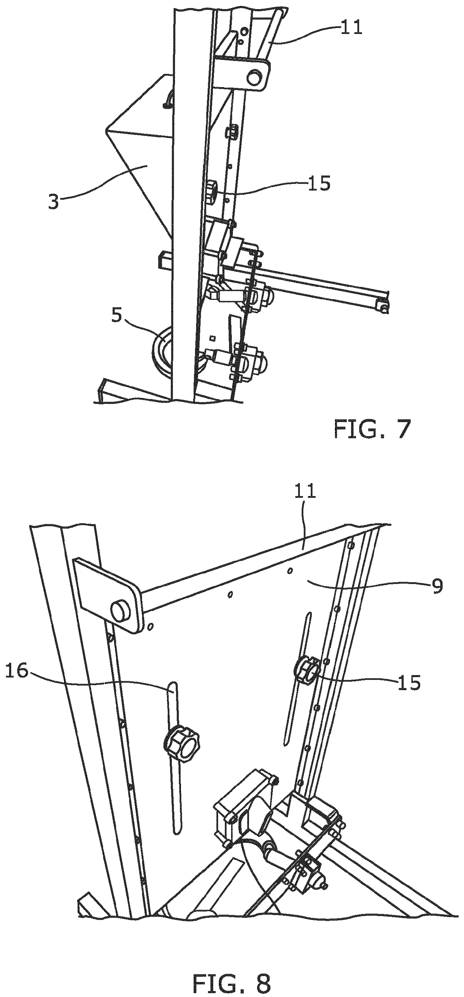

The frame (2) comprises two upright members (6) which extend from the base (1). A planar element (7) is mounted substantially vertically on the frame. The planar element (7) has a front face (8) and a rear face (9), the front face being the face which faces the user when the device is in use. The element (3) is adjustably mounted on the front face (8) of the planar element (7). The element (3) can slide up and down on the frame (2) within two slots (16) and can be locked in position via the two lock wheels (15) as illustrated in the Figs 7 and 8. Suitably the inclined surface (4) of the element (3) is inclined at a 30° angle to the horizontal such that its slopes away from the user towards the base (1) of the device. The inclined surface (4) may be provided with padded material. The upper in use surface of the element (3) is provided with a handle (10) which can be used to move the element up and down in relation to the frame, thus adjusting the height of the element on the planar element (7).

The two hook shaped elements (5) are attached to a bar (11) located at the base (2) of the device and fixed between the two upright members (6). Each hook shaped element (5) is provided with a force sensor which is a loadcell(s). Typically there is one loadcell in each hook, but that could be varied. The loadcell can convert the force applied by the ankles of the user into a measurable electrical output. The electrical output is transmitted to a software platform by Wi-Fi and the software platform can record and analyse that output.

The hook shaped elements (5) are shaped so that they can be fastened around the ankle of the user. This may be achieved by means of a strap (not shown), or different sizes of hook shaped elements (5) can be provided with the device. The hook shaped element may be made of a plastics material so it is easy to wipe clean sterilise. Suitable hook shaped elements are commercially available at least from VALD Performance, Australia.

The device is provided with a handle (12) that the user can grasp during use of the device. The handle (12) is in the form of a bar positioned along the upper, in use position, of the device. The handle (12) is fixed at either end to one of the upright members (2). Other handle structures are possible, such as two handgrips, one positioned on either side of the device, towards the upper in use position.

The base (2) is generally U-shaped with two elongate elements (13) which are connected by another elongate element (14) adjacent their ends. The upright members (2) extend upwardly from the elongate elements (13). However, other suitable base configurations are possible.

Generally, the base (2) and the upright members (3) are made from a heavy, durable material such as metal, so that the device cannot be easily knocked over when it is being used.

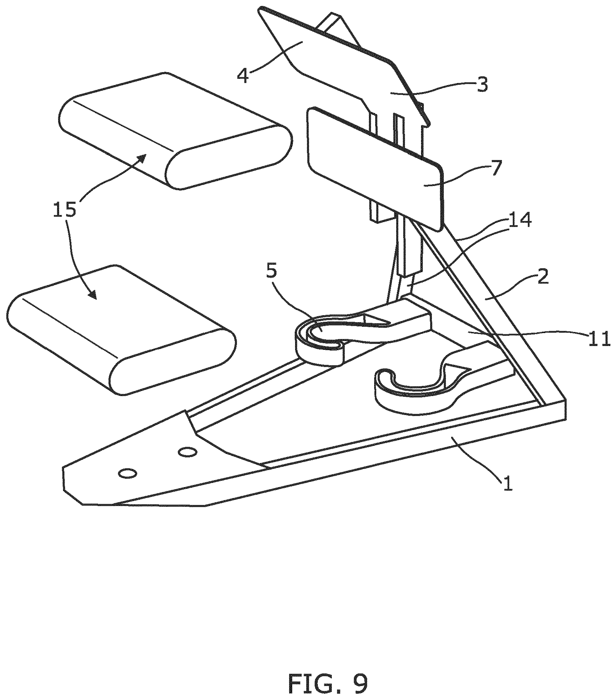

A second embodiment of the invention is shown in Figure 9. It also a base (1) adapted to sit on the ground, a frame (2) which extends vertically from the base, a thigh support element (3) having an inclined surface (4) which is adjustably mounted on the frame (2) and two hook shaped elements (5) mounted adjacent the base (1) of the device.

The frame (2) comprises two upright members (14) which extend from the base (1). A planar element (7) is mounted substantially vertically on the frame. The element (3) is adjustably mounted on the frame (2). The element (3) can slide up and down on the frame (2) and can be locked in position. Suitably the inclined surface (4) of the element (3) is inclined at a 30° angle to the horizontal such that its slopes away from the user towards the base (1) of the device. The inclined surface (4) may be provided with padded material.

The two hook shaped elements (5) are attached to a bar (11) located at the base (2) of the device and fixed between the two upright members (14). Each hook shaped element (5) is provided with a force sensor which is a loadcell(s).

The device is provided with an adjustable seat with the figure showing suitable alternative heights for the seat depending on which measurement is being made.

The method of using the device and analysing hamstring function will now be described using the H-rig which is the device of the invention.

### Example

### 1. Testing procedures

**Bi-Lateral H-Rig Test** - Iso_{BI} 0.89, ICC=0.79-0.94.
∘ The participant's knees should rest comfortably against the pad while the ankles (malleoli) are positioned directly in line vertically with the knee.
∘ The participant should be placed in a seated position.
∘ The participant's feet should rest on the floor at the start position.
∘ Once the participant achieves a comfortable position, they should be seated 30cm away from the pad.
∘ The participant should be seated at a height that helps to achieve 30 degrees of knee flexion, by adjusting the seat height and feet positioned on the ground.
∘ 30 degrees of knee flexion is achieved by sliding the 30 degree thigh support element into place and locking it to hold the knee in 30 degrees of knee flexion.
∘ The participant's trunk should remain upright with both arms crossed in front of their chest.
∘ The participant should be queued to drive their knees into the thigh support element while each ankle should drive posteriorly into the ankle hooks.
∘ Three maximal repetitions should be carried out with the maximal force output being recorded.

**Uni-Lateral H-Rig Test** - Iso_{UNI} (0.9, ICC=0.8-0.95)
∘ The participant should ensure that their trunk is in an upright position throughout test.
∘ Both arms should remain fully locked out and should be placed on the horizontal grip bar of the H-Rig.
∘ A 50cm high bench should be placed 60cm away from the face of the H-Rig.
∘ 20 degrees of hip extension in the contralateral (non-tested limb) should be measured using a goniometer.
∘ If this angle needs to be adjusted to find 20 degrees of extension, the bench can be moved towards or away from the rig to do so.
∘ The un-involved knee should be positioned on the bench or seat, with the patella resting at its nearest point.
∘ The contralateral limb is then positioned with the patella placed against the H-Rig.
∘ A 30 degree thigh support element is then placed between the anterior thigh and the rig in order to form a 30 degree angle of knee flexion.
∘ Three sets of two maximal reps should take place, with the involved limb alternating between sets.
∘ Repetitions should last for five seconds.
∘ Five minutes of recovery should be allowed before carrying out the eccentric strength test.

### 2. Clinical Research

A total of 70 amateur Gaelic Football players with 18 previous HSI were tested in the preseason period (January to March).

| | Iso_{BI} | | |
|---|---|---|---|
| | **Involved** | **Non-Involved** | **Effect size** |
| **Absolute Force (N)** | 281±66 | 332±72 | 0.738 |
| **Relative force (N.kg)** | 3.48±0.89 | 4.11±0.92 | 0.696 |
| **Absolute Torque (Nm)** | 120±29 | 142±31 | 0.72 |
| **Relative Torque (Nm.kg)** | 1.49±.39 | 1.76±.40 | 0.683 |
| Scaled Force | 5.2±1.22 | 6.14±1.33 | 0.736 |

### Previously injured isometric hamstring variables (P>0.05)

Iso_{BI} force and torque all differed significantly with a moderate effect size (p<0.01, d=0.68-0.74) between the injured and uninjured sides in players with previous hamstring injury with bilateral deficits of +15% which also included players who suffered HSI in the previous season. This indicates that strength deficits still exist and it may be that Iso_{BI} testing is more sensitive to detecting residual or underlying weaknesses as it assesses muscle function in a long lever 150° position.

A total of 49 amateur Gaelic Football (26.5+/-2.4yrs; 81.6+/-9.1 kgs) were tested in the preseason period (June) of whom sustained a previous hamstring strain in the past 12 months with mean time lost per HSI to training or matches 20.1±7.1 days. The major mechanism was high speed running and kicking, accounting for 89% and 11% of hamstring strains, respectively. In Iso_{UNI} there were significant differences between the injured and non-injured group for absolute force, relative force, scaled force, absolute torque, scaled torque and relative torque measures (p>0.05).

| | **Injured** | **Non-injured** | **ES** |
|---|---|---|---|
| **Ab Force (N)** | 363±62 | 417±72 | 0.80 |
| **Relative force (N.kg)** | 4.48±0.85 | 5.19±0.86 | 0.92 |
| **Scaled force** | 6.68±1.26 | 7.75±1.28 | 0.92 |
| **Ab Torque (Nm)** | 156±28 | 185±34 | 0.93 |
| **Relative Torque (Nm.kg)** | 1.92±0.38 | 2.33±0.39 | 1.06 |
| **Scaled Torque** | 1.88±0.37 | 2.28±0.38 | 1.06 |

Comparison of Iso_{UNI} variables from those with previous HSI injured versus non-injured players (mean of L+R) (*P>0.01)*

As we have indicated Iso_{BI} strength better identifies those with residual strength deficits following HSI in the previous season and in this current cohort there is trend towards a Iso_{UNI} underlying weakness in previously injured players. Iso_{UNI} may be more sensitive to underlying residual deficits as this long lever position may more closely simulate the mechanism of injury seen in late swing phase/early stance in which the contralateral limb is also in extension. The test is unilateral preventing any neural crossover isolating any potential weaknesss. Also in Iso_{UNI} the opposite hip is placed into extension and subjective feedback from participants indicates this places a counter stretch on the limb been tests and perhaps even encourages a "cocking mechanism" prior to MVIC.

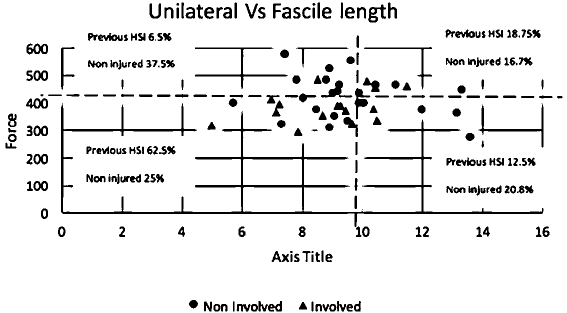

Fascicle length in the injured group was moderately correlated with Iso_{BI} (r=0.442, P< 0.086), Iso_{UNI} (r=0.389, P< 0.136). 62.5% of all previous hamstring injuries scored below the averages of the group for both fascicle length (9.9cm) and unilateral isometric strength (420N). Our results suggest that Iso_{UNI} and Iso_{BI} is related to BFlh *L*_{f} in previously injured Gaelic footballers. The main advantages of these isometric tests is 1) the Iso_{BI} assesses muscle function in a long lever 150⁰ position 2) The Iso_{UNI} may isolate the affected muscle group and preclude any crossover between limbs which occurs in bilateral testing, secondly the placement of the contralateral hip into extension may simulate the mechanism of injury in which the contralateral hip is in extension during late swing phase/early stance as running is the main mechanism of injury. The isometric testing may expose the vulnerability for injury by testing in a position in which the hamstrings are in their position of greatest risk and may help explain the disparity between eccentric and isometric testing and provide an insight to the greater relationship to BFlh *L*_{f}.

A total of 30 HSI were tested 0-7days following acute hamstring injury. 30 club footballers who presented following an acute onset of posterior thigh pain in either training or competition were invited to participate in the study. These players were unable to train or participate in Gaelic football at the time of their injury. The initial hamstring injury diagnosis was made by a lead clinician and verified by a clinical colleague. Hamstring injury was defined as a posterior thigh injury, to the hamstring muscle group and were indirect muscle disorders of the musculotendinous complex of biceps femoris, semitendinosus and semimembranosus. Injuries were graded according to the signs and symptoms as described by (Pollock et al., 2014). The myofascial/musculotendinous/intratendinous component of the classification system was omitted and injuries referred to as Grade 0, I, II, III and IV, as MRI is used to determine the extent or component of HSI. Inspection and palpation, active knee extension, passive straight leg raise, manual muscle testing and VAS were all used to corroborate the diagnosis.

In the bilateral isometric test there was no difference (p>0.05) between the involved and uninvolved sides for Grade 0 (224±42v246±59N), Grade I (204±67v 240±49N), and II (137±56v 163±36N), respectively. The ratios were 0.94±0.19, 0.86±0.26 and 0.84±0.25 for Grade 0, I and II. In the unilateral isometric test there were significant differences (p<0.05) between the involved and uninvolved sides for Grade 0 (351±99v 402±74N), II (252±101v 380±99N), and III (187±62v327±58N), receptively. The involved limb ratios were 0.87±0.17, 0.66±0.18 and 0.59±0.20 for Grade 0, I and II.

| | **Involved side** | **Un-Involved side** | **Ratio** | **P-Value** |
|---|---|---|---|---|
| **Grade 0** | 351±99 | 402±74 | 0.87±0.17 | 0.043 |
| **Grade I** | 252±101 | 380±99 | 0.66±0.18 | 0.003 |
| **Grade II** | 187±62 | 327±58 | 0.59±0.20 | 0.016 |

Unilateral isometric strength for involved versus uninvolved side (p<0.05)

| **Bilateral Isometrics** | | | **Uni-lateral Isometrics** | | |
|---|---|---|---|---|---|
| | **Total** | | | **Total** | |
| | **N** | % | | **N** | % |
| **Positive** | 27 | 57 | **Positive** | 24 | 80 |
| **Negative** | 13 | 43 | **Negative** | 6 | 20 |
| **Total** | 30 | 100 | **Total** | 30 | 100 |

Clinical assessment and <10% bilateral deficit in isometrics strength and hamstring strain injury

Level of agreement between clinical hamstring assessment and <10% bilateral deficit in isometric strength with regard to the presence of injury. In 27 cases (57%) there was a greater than 10% deficit in bilateral isometric strength with associated hamstring injury. In 24 (80%) of the cases there was a greater than 10% deficit in unilateral isometric strength with associated hamstring injury.

| | **Grade r^{a}** | **P** |
|---|---|---|
| **Unilateral Iso Test** | 0.553 | <0.01 |
| **Bilateral Iso Test** | 0.350 | <0.06 |
| **Bilateral Ratios** | 0.163 | <0.04 |
| **Unilateral ratios** | 0.587 | <0.01 |

Correlation between clinical assessment of hamstring injury and isometric strength

### ^{a} Spearman rank correlation

The isometric strength during the clinical assessment injury was highly correlated with unilateral isometric strength and the grade of hamstring injury (r=0.002, P< 0.01) and moderate correlations for the bilateral test was (r=0.058, P<0.06). The bilateral ratio during the clinical assessment injury was highly correlated with unilateral isometric ratio and the grade of hamstring injury (r=0.587, P< 0.01) and low correlations for the bilateral test (r=0.163, P<0.04).

| | Bilateral | Unilateral |
|---|---|---|
| Grade 0 | 0.94±0.17 | 0.88±0.18 |
| Grade I | 0.86±0.26 | 0.66±0.18 |
| Grade II | 0.84±0.25 | 0.59±0.20 |

Bilateral Ratio and grading of hamstring injury

The ISOᵤₙᵢ of the involved side was significantly weaker in comparison to the uninjured limb for grade 0, I and II HSI classification, and a high level of agreement and correlation between ISOᵤₙᵢ ratios and clinical assessment in the classification of HSI. Iso_{UNI} ratios (0-7 days post injury) can provide an indication of HSI grade and classification and we suggest Iso_{UNI} ratios of 0.9, 0.7, 0.6 to corroborate clinical diagnosis and grading of 0, I and II hamstring injuries respectively.

### 3. Normative Data and Recommendations

| | ISO_{BI} | ISOᵤₙᵢ | |
|---|---|---|---|
| **Previous Injury** | <15% deficit | | We recommend a 15% deficit in screening can be indicative of previous HSI in the past season. |
| | | | |
| **Future Injury** | 150Nm | 185Nm | We recommend cut off points of 150 Nm for the bilateral test and 185Nm for unilateral test to protect from future injury. |
| | | | |
| **Classification ratio** | | | |
| **Grade 0** | 0.95 | 0.87 | We recommend from our research that opposite limb ratios in the unilateral test, where possible be widely used to assist in the classification of HSI. |
| **Grade I** | 0.86 | 0.66 | |
| **Grade II** | 0.84 | 0.59 | |
| | | | |
| **Strength Classification** | | | |
| **Good** | 320+ | 505+ | **Green represents a good score which reduces the risk of hamstring injury, athlete should** |
| | | | **continue to work on increasing hamstring strength** |
| **Average** | 250-319 | 435-505 | **Orange represents a score around the average strength of squad of players and the Athletes should work on increasing strength into the green category** |
| **Poor** | -250 | -435 | **Red represents any score below this average threshold or a 10% bilateral difference from left to right. Athlete needs to work to increase the hamstring strength in this catergory** |
| | | | |

### 4. Return to play programming and Recommendations

**Return to Play Timelines for Grade 0, Grade I & Grade II Hamstring Injuries. Grade 0**: Recovery Time; 17±10 days.

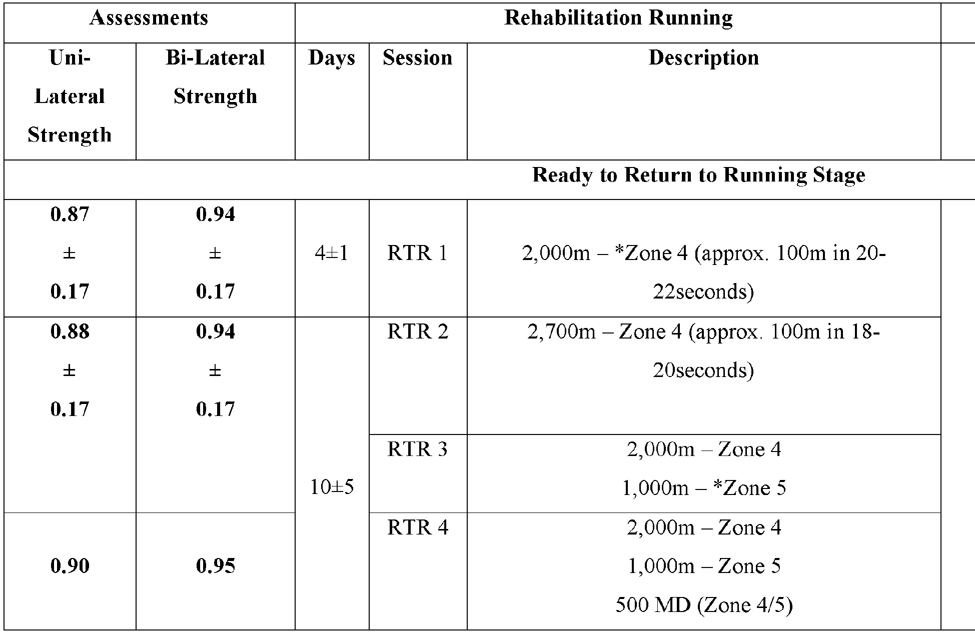

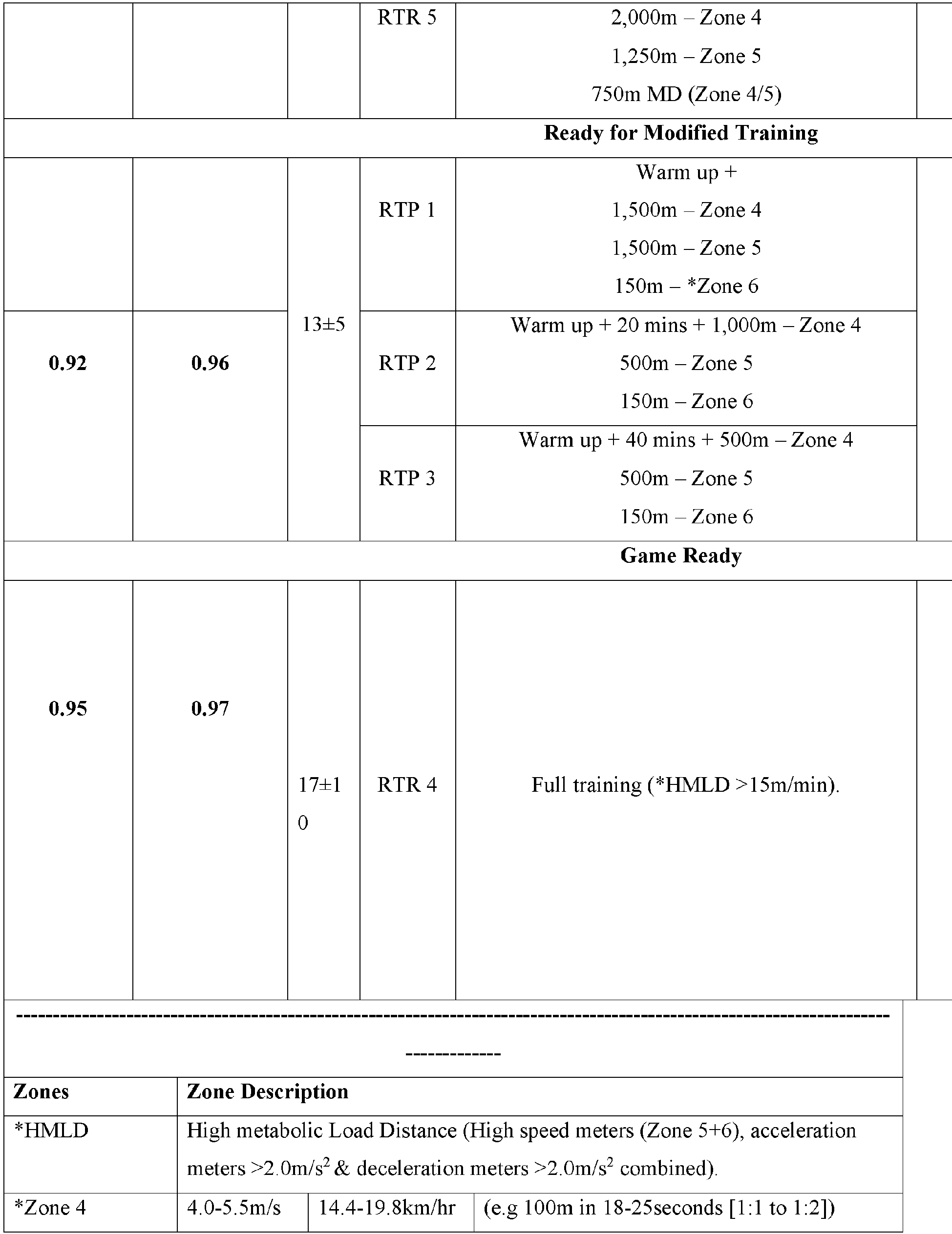

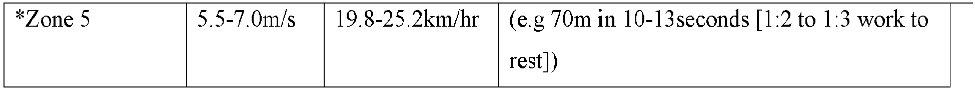

The words "comprises/comprising" and the words "having/including" when used herein are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features which are, for brevity, described in the context of a single embodiment, may also be provided separately or in any suitable sub-combination.

## Claims

1. A device for the measurement of hamstring function comprising a base (1) adapted to sit on the ground, a frame (2) extending vertically from the base (1) having two slots, a thigh support element (3) having an inclined surface (4) inclined at 27 to 33° to the horizontal adjustably mounted on the frame (2) such that the element (3) can slide up and down on the frame (2) within the two slots (16) and be locked in position via two lock wheels (15) and at least one hook shaped element (5) which can be fastened around the ankle of a user, the hook shaped element (5) being mounted adjacent to the base (1) of the device, each of the hook shaped elements comprising at least one force sensor which can sense the force applied about the knee through the ankle by one of the hamstring of a user pushing their thighs against the thigh support element (3), and which can transmit the force which is sensed to a device which can record and measure the force.

2. A device as claimed in claim 1 wherein the frame (2) comprises a planar element (7) which is mounted substantially vertically on the frame.

3. A device as claimed in claim 2 wherein the planar element (7) has a front face (8) and a rear face (9), the thigh support element (3) being adjustably mounted on the front face (8) of the planar element (7).

4. A device as claimed in any preceding claim wherein the inclined surface (4) of the thigh support element (3) is inclined at 29 to 31° to the horizontal.

5. A device as claimed in claim 4 wherein the inclined surface (4) of the thigh support element (3) is inclined at 30° to the horizontal.

6. A device as claimed in any preceding claim wherein the force sensor is a loadcell.

7. A device as claimed in any preceding claim wherein the device is provided with two hook shaped elements, one for each ankle of the user.

8. A device as claimed in any preceding claim wherein the device is provided with a handle (10) that the user can grasp during use of the device.

9. A device as claimed in any preceding claim further comprising a seat.

10. A device as claimed in claim 9 wherein the height of the seat is adjustable.

11. A method of measuring bilateral hamstring function using the device of claim 7 or claims 8-10 depending on claim 7, comprising sensing and measuring the force applied by the ankles of a user, the user being in a seated position and pushing their thighs against a thigh support element (3) having an inclined surface (4) inclined at 30° to the horizontal when the knees are in 150° extension, and the force about the knee being measured through the ankle by a force sensor attached to the ankles.

12. A method of measuring unilateral hamstring function with the tested leg using the device of any of claims 1 to 10, comprising sensing and measuring the force applied by the ankles of a user, the user being in an upright position and pushing the thighs of the tested leg against a thigh support element (3) having an inclined surface (4) inclined at 30° to the horizontal with the non-tested leg in 10-30° of hip extension when the knees are in 150° extension, and the force about the knee being measured through the ankle by a force sensor attached to the ankles.

## Patentansprüche

1. Eine Vorrichtung für die Messung der Hamstring-Funktion, die Folgendes aufweist:
eingerichtet zum Stehen auf dem Boden, einen Rahmen (2), der sich vertikal von der Basis (1) erstreckt und zwei Schlitze hat, ein Oberschenkel-Stützelement (3), das eine geneigte Fläche (4) hat, die um 27 bis 33° zur Horizontalen geneigt ist, verstellbar an dem Rahmen (2) montiert, derart, dass das Element (3) an dem Rahmen (2) innerhalb der zwei Schlitze (16) auf und ab gleiten und über zwei Arretierräder (15) in einer Position arretiert werden kann, und wenigstens ein hakenförmiges Element (5), das um den Knöchel eines Benutzers befestigt werden kann,
wobei das hakenförmige Element (5) angrenzend an die Basis (1) der Einrichtung montiert ist, wobei jedes der hakenförmigen Elemente wenigstens einen Kraftsensor aufweist, der die um das Knie durch den Knöchel aufgebrachte Kraft erfassen kann, die vom Hamstring eines Benutzers aufgebracht wird, der seine Oberschenkel gegen das Oberschenkel-Stützelement (3) drückt, und der die erfasste Kraft an eine Einrichtung übertragen kann, die die Kraft aufzeichnen und messen kann.

2. Eine Einrichtung nach Anspruch 1, wobei der Rahmen (2) ein planares Element (7) aufweist, das im Wesentlichen vertikal an dem Rahmen montiert ist.

3. Eine Einrichtung nach Anspruch 2, wobei das planare Element (7) eine Vorderseite (8) und eine Rückseite (9) hat, wobei das Oberschenkel-Stützelement (3) verstellbar an der Vorderseite (8) des planaren Elements (7) montiert ist.

4. Eine Einrichtung nach einem vorhergehenden Anspruch, wobei die geneigte Fläche (4) des Oberschenkel-Stützelements (3) um 29 bis 31° zur Horizontalen geneigt ist.

5. Eine Einrichtung nach Anspruch 4, wobei die geneigte Fläche (4) des Oberschenkel-Stützelements (3) um 30° zur Horizontalen geneigt ist.

6. Eine Einrichtung nach einem vorhergehenden Anspruch, wobei der Kraftsensor eine Kraftmessdose ist.

7. Eine Einrichtung nach einem vorhergehenden Anspruch, wobei die Einrichtung mit zwei hakenförmigen Elementen versehen ist, eines für jeden Knöchel des Benutzers.

8. Eine Einrichtung nach einem vorhergehenden Anspruch, wobei die Einrichtung mit einem Griff (10) versehen ist, den der Benutzer während der Verwendung der Einrichtung ergreifen kann.

9. Eine Einrichtung nach einem vorhergehenden Anspruch, ferner einen Sitz aufweisend.

10. Eine Einrichtung nach Anspruch 9, wobei die Höhe des Sitzes einstellbar ist.

11. Ein Verfahren zur Messung der bilateralen Hamstring-Funktion unter Verwendung der Einrichtung nach Anspruch 7 oder den Ansprüchen 8-10 in Abhängigkeit von Anspruch 7, das Folgendes aufweist: Erfassen und Messen der durch die Knöchel eines Benutzers aufgebrachten Kraft, wobei der Benutzer sich in einer Sitzposition befindet und seine Oberschenkel gegen ein Oberschenkel-Stützelement (3) drückt, das eine geneigte Fläche (4) hat, die um 30° zur Horizontalen geneigt ist, wenn sich die Knie in 150° Extension befinden, und wobei die Kraft um das Knie durch den Knöchel von einem an den Knöcheln befestigten Kraftsensor gemessen wird.

12. Ein Verfahren zur Messung der unilateralen Hamstring-Funktion mit dem getesteten Bein unter Verwendung der Einrichtung nach einem der Ansprüche 1 bis 10, das Folgendes aufweist: Erfassen und Messen der durch die Knöchel eines Benutzers aufgebrachten Kraft, wobei der Benutzer sich in einer aufrechten Position befindet und die Oberschenkel des getesteten Beins gegen ein Oberschenkel-Stützelement (3) drückt, das eine geneigte Fläche (4) hat, die um 30° zur Horizontalen geneigt ist, wobei das nicht-getestete Bein in 10-30° Hüftextension ist, wenn sich die Knie in 150° Extension befinden, und wobei die Kraft um das Knie durch den Knöchel von einem an den Knöcheln befestigten Kraftsensor gemessen wird.

## Revendications

1. Dispositif de mesure de la fonction des ischio-jambiers comprenant une base (1) adaptée pour reposer sur le sol, un cadre (2) s'étendant verticalement à partir de la base (1) présentant deux fentes, un élément de support de cuisse (3) présentant une surface inclinée (4) inclinée de 27 à 33° par rapport à l'horizontale monté réglable sur le cadre (2) de sorte que l'élément (3) puisse coulisser vers le haut et vers le bas sur le cadre (2) à l'intérieur des deux fentes (16) et être verrouillé en position par deux roues de verrouillage (15) et au moins un élément en forme de crochet (5) qui peut être fixé autour de la cheville d'un utilisateur, l'élément en forme de crochet (5) étant monté adjacent à la base (1) du dispositif, chacun des éléments en forme de crochet comprenant au moins un capteur de force qui peut détecter la force appliquée au niveau du genou à travers la cheville par l'un des ischio-jambiers d'un utilisateur poussant ses cuisses contre l'élément de support de cuisse (3), et qui peut transmettre la force qui est détectée à un dispositif qui peut enregistrer et mesurer la force.

2. Dispositif selon la revendication 1, dans lequel le cadre (2) comprend un élément plan (7) qui est monté sensiblement verticalement sur le cadre.

3. Dispositif selon la revendication 2, dans lequel l'élément plan (7) présente une face avant (8) et une face arrière (9), l'élément de support de cuisse (3) étant monté de manière réglable sur la face avant (8) de l'élément plan (7).

4. Dispositif selon l'une quelconque des revendications précédentes, dans lequel la surface inclinée (4) de l'élément de support de cuisse (3) est inclinée de 29 à 31° par rapport à l'horizontale.

5. Dispositif selon la revendication 4, dans lequel la surface inclinée (4) de l'élément de support de cuisse (3) est inclinée à 30° par rapport à l'horizontale.

6. Dispositif selon l'une quelconque des revendications précédentes, dans lequel le capteur de force est une cellule de charge.

7. Dispositif selon l'une quelconque des revendications précédentes, dans lequel le dispositif est doté de deux éléments en forme de crochet, un pour chaque cheville de l'utilisateur.

8. Dispositif selon l'une quelconque des revendications précédentes, dans lequel le dispositif est doté d'une poignée (10) que l'utilisateur peut saisir pendant l'utilisation du dispositif.

9. Dispositif selon l'une quelconque des revendications précédentes comprenant en outre un siège.

10. Dispositif selon la revendication 9, dans lequel la hauteur du siège est réglable.

11. Procédé de mesure de la fonction bilatérale des ischio-jambiers à l'aide du dispositif selon la revendication 7 ou selon les revendications 8 à 10 quand elles dépendent de la revendication 7, comprenant la détection et la mesure de la force appliquée par les chevilles d'un utilisateur, l'utilisateur étant en position assise et poussant ses cuisses contre un élément de support de cuisse (3) présentant une surface inclinée (4) à 30° par rapport à l'horizontale lorsque les genoux sont en extension de 150°, et la force autour du genou étant mesurée à travers la cheville par un capteur de force fixé aux chevilles.

12. Procédé de mesure unilatérale de la fonction des ischio-jambiers avec la jambe testée à l'aide du dispositif selon l'une quelconque des revendications 1 à 10, comprenant la détection et la mesure de la force appliquée par les chevilles d'un utilisateur, l'utilisateur étant en position verticale et poussant les cuisses de la jambe testée contre un élément de support de cuisse (3) présentant une surface inclinée (4) inclinée à 30° par rapport à l'horizontale avec la jambe non testée en extension de hanche de 10 à 30° lorsque les genoux sont en extension à 150°, et la force sur le genou est mesurée à travers la cheville par un capteur de force fixé aux chevilles.
